(19) Europäisches Patentamt
European Patent Office
Office européen des brevets

(11) **EP 2 157 442 B1**

(12) **EUROPEAN PATENT SPECIFICATION**

(45) Date of publication and mention
of the grant of the patent:
**09.10.2013 Bulletin 2013/41**

(51) Int Cl.:
***G01S 7/52*** *(2006.01)*

(21) Application number: **09167774.0**

(22) Date of filing: **13.08.2009**

(54) **Formation of an elastic image in an ultrasound system**

Bildung eines elastischen Bildes in einem Ultraschallsystem

Formation d'une image élastique dans un système à ultrasons

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.08.2008 KR 20080082181**

(43) Date of publication of application:
**24.02.2010 Bulletin 2010/08**

(73) Proprietor: **Samsung Medison Co., Ltd.**
**Gangwon-do (KR)**

(72) Inventors:
• **Shin, Dong Kuk**
**135-851 Seoul (KR)**
• **Jeong, Mok Kun**
**139-768 Seoul (KR)**

(74) Representative: **Schmid, Wolfgang**
**Lorenz & Kollegen**
**Patentanwälte Partnerschaftsgesellschaft**
**Alte Ulmer Strasse 2**
**89522 Heidenheim (DE)**

(56) References cited:
**EP-A1- 1 520 517          FR-A1- 2 899 336**
**US-A1- 2007 073 145     US-B1- 6 558 324**

• **DUMONT ET AL: "ARFI imaging for noninvasive material characterization of atherosclerosis" ULTRASOUND IN MEDICINE AND BIOLOGY, NEW YORK, NY, US, vol. 32, no. 11, 14 November 2006 (2006-11-14), pages 1703-1711, XP005767689 ISSN: 0301-5629**
• **BEHLER R H ET AL: "ARFI Ultrasound for Characterizing Atherosclerosis" ULTRASONICS SYMPOSIUM, 2006. IEEE, IEEE, PI, 1 October 2006 (2006-10-01), pages 722-727, XP031076367 ISBN: 978-1-4244-0201-4**
• **FAHEY ET AL: "Acoustic radiation force impulse imaging of the abdomen: demonstration of feasibility and utility" ULTRASOUND IN MEDICINE AND BIOLOGY, NEW YORK, NY, US, vol. 31, no. 9, 1 September 2005 (2005-09-01), pages 1185-1198, XP005081489 ISSN: 0301-5629**
• **ZHAI L ET AL: "Visualizing the Anatomic Structures of Human Prostates Using Acoustic Radiation Force Impulse (ARFI)Imaging" ULTRASONICS SYMPOSIUM, 2007. IEEE, IEEE, PISCATAWAY, NJ, USA, 1 October 2007 (2007-10-01), pages 432-435, XP031195008 ISBN: 978-1-4244-1383-6**

## Description

### TECHNICAL FIELD

The present disclosure relates to ultrasound systems, and more particularly to the formation of an elastic image in an ultrasound system.

### BACKGROUND

Recently, an ultrasound system has been extensively used in the medical field due to its non-invasive and non-destructive nature. Modern high-performance ultrasound imaging systems and techniques are commonly used to produce two dimensional ultrasound images and three-dimensional ultrasound images of internal features of patients.

Generally, the ultrasound image is displayed in a Brightness mode (B-mode) by using reflectivity caused by an acoustic impedance difference between the tissues of a target object. However, if the reflectivity of the target object is hardly different from those of the neighboring tissues such as tumor, cancer or the like, then it is not easy to recognize the target object in the B-mode image. Further, an ultrasound elastic imaging technology has been developed to display an image of the target object by using mechanical characteristics of the target object. Such technology is very helpful for diagnosing lesions such as cancers. The tumor or cancer is relatively stiffer than the neighboring tissues. Thus, when a pressure is uniformly applied, a variation of the tumor or cancer is typically smaller than those of the neighboring tissues.

The elasticity of a tissue is measured by using ultrasound data obtained before and after applying the pressure to the tissue. A compression plate mounted on an ultrasound probe may be used to apply the pressure to the tissue. A user may press the compression plate on the target object, thereby applying the pressure to the tissues of the target object. In such a case, strain data in the tissues may be varied according to the pressure applied by the user. Thus, the video quality of an elastic image may be changed according to the pressure applied to the tissue.

FR 2 899 336 A1 discloses a method for performing excitation during which an internal mechanical constraint e.g. vibration, is generated in an excitation zone. Imaging is performed by acquiring signals during movements generated in a viscoelastic medium e.g. ovary, based on the constraint in an imaging/observation zone. An quantitative index related to rheological properties is calculated in a point of the zone situated at a depth outside the zone, where the index represents a comparison of the signals acquired during movements generated by a point of the zone based on the constraint.

Documents XP005767689 "ARFI imaging for noninvasive material characterization of atherosclerosis" by Douglas Dumont et al., XP031076367 "ARFI ultrasound for characterizing atherosclerosis" by R.H. Behler et al. and XP005081489 "Acoustic radiation force impulse imaging of the abdomen: Demonstration of feasibility and utility" by R.H. Behler et al. describe causes of and measures against cardiovascular disease (CVD).

A method for estimating tissue velocity vectors and oriented strain from ultrasonic diagnostic imaging data is known from EP 1 520 517 A1. The method comprises the following steps: Acquiring ultrasound imaging data from an object by transmitting ultrasound beams against the object and receiving the corresponding reflected beams by the object; defining at least one or a certain number of reference points in an ultrasonic image field corresponding to the ultrasonic image data obtained from evaluation of the ultrasonic reflected beams; determining the direction of motion and the velocity vector of the reference points from the ultrasonic image data. The ultrasound imaging data consist in a sequence of at least two image frames, said images being two dimensional images or three dimensional data. The imaging data are B-mode grey-scale echographic images. The velocity of motion of each reference point between two successive B-mode image frames are determined by applying a so called particle image velocimetry technique abbreviated as PIV.

US 2007/073145A1 refers to a method for elasticity ultrasound imaging. According to the method, using compression, tissue elasticity data from two or more different fields of view is acquired. Since different amounts of compression may be used for the different fields of view, the dynamic range of the elasticity data is updated. A panoramic elasticity image is generated from the updated elasticity data of multiple fields of view. A panoramic elasticity image represents the combined fields of view for the elasticity data.

A system and method for strain image display are disclosed in US 6,558,324 B1. A region of interest (ROI) of a patient's body is repeatedly scanned using an ultrasound transducer array. Data sets representing an image property, such as intensity, from portions of the ROI are used to calculate a representation of the displacement profile within the ROI at different stress levels. From the displacement profile, a function of strain is also preferably calculated.

### SUMMARY

Embodiments for forming an elastic image in an ultrasound system are disclosed herein.

According to the invention, an ultrasound system comprises the features contained in claim 1.

Furthermore, a method of forming an elastic image in an ultrasound system comprises the features according to claim 5

Still further, a computer readable medium comprises the features of claim 9.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

## BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit within the ultrasound system as shown in FIG. 1.
FIG. 3 is a block diagram showing an illustrative embodiment of a processing unit within the ultrasound system as shown in FIG. 1.
FIG. 4 is a schematic diagram showing an example of feature points.

## DETAILED DESCRIPTION

A detailed description may be provided with reference to the accompanying drawings. One of ordinary skill in the art may realize that the following description is illustrative only and is not in any way limiting.

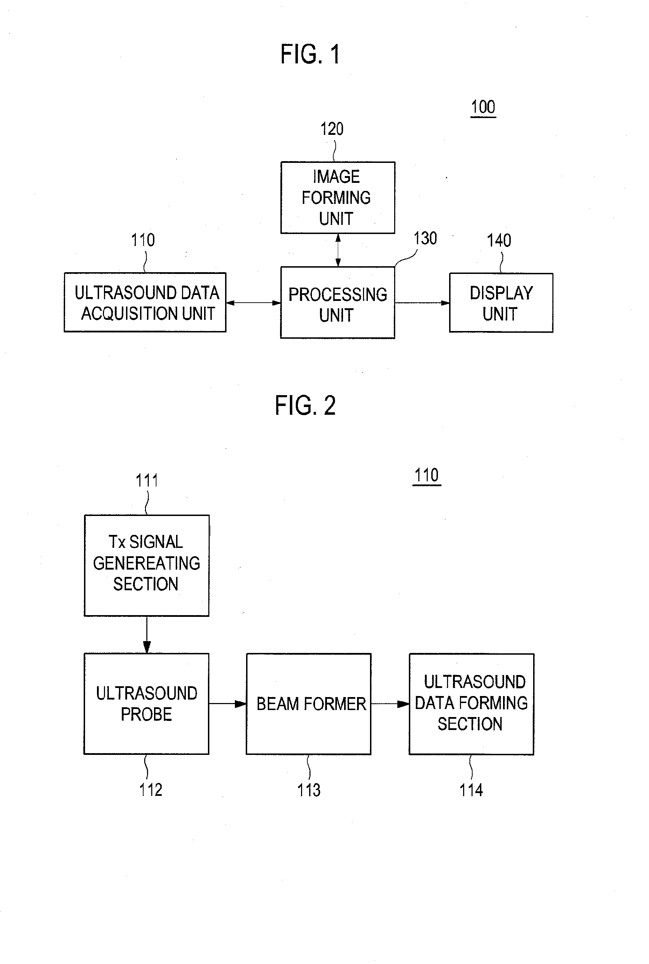

Referring to FIG. 1, an ultrasound system 100 in accordance with an illustrative embodiment is shown. As depicted therein, the ultrasound system 100 includes an ultrasound data acquisition unit 110 configured to transmit/receive ultrasound signals to/from a target object to thereby acquire ultrasound data. The ultrasound data acquisition unit 110 includes a transmit (Tx) signal generating section 111, as shown in FIG. 2.

Referring to FIG. 2, the Tx signal generating section 111 is operable to generate first Tx signals. The Tx signal generating section 111 is further operable to generate second Tx signals in consideration of first feature points of the target object in a reference image. The first feature points and the reference image are defined as below.

The ultrasound data acquisition unit 110 further includes an ultrasound probe 112 containing a plurality of elements for reciprocally converting between ultrasound signals and electrical signals. The ultrasound probe 112 is operable to transmit first ultrasound signals to the target object in response to the first Tx signals. The ultrasound probe 112 is further operable to receive echo signals reflected from the target object to thereby output first received signals. The ultrasound probe 112 is further operable to transmit second ultrasound signals for applying an acoustic radiation force impulse (ARFI) to the target object in response to the second Tx signals so that the compression to the target object can be performed. The second ultrasound signals are focused on each of the first feature points. The ultrasound probe 112 is also operable to receive echo signals reflected from the target object to thereby output second received signals.

The ultrasound data acquisition unit 110 further includes a beam former 113. The beam former 113 is operable to convert the first received signals into first digital signals. The beam former 113 is further operable to apply delays to the first digital signals in consideration of a distance between the elements and focal points to thereby output first digital receive-focused signals. The beam former 113 is operable to convert the second received signals into second digital signals. The beam former 113 is further operable to apply delays to the second digital signals in consideration of a distance between the elements and focal points (i.e., first feature points) to thereby output second digital receive-focused signals.

The ultrasound data acquisition unit 110 further includes an ultrasound data forming section 114. The ultrasound data forming section 114 is operable to form first ultrasound data based on the first digital receive-focused signals. The ultrasound data forming section 114 is further operable to form second ultrasound data based on the second digital receive-focused signals.

Referring back to FIG. 1, the ultrasound system 100 further includes an image forming unit 120. The image forming unit 120 is operable to form the reference image based on the first ultrasound data. The reference image includes one of a brightness mode (B mode) image and a three-dimensional image. The image forming unit 120 is further operable to form an ARFI image based on the second ultrasound data.

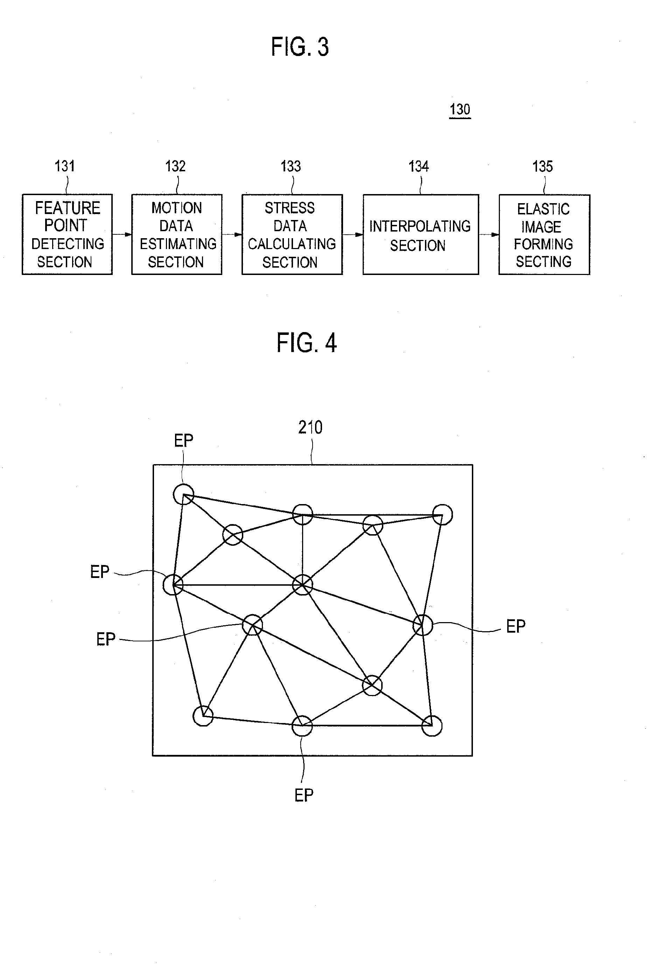

The ultrasound system 100 further includes a processing unit 130 that is connected to the ultrasound data acquisition unit 110 and the image forming unit 120. The processing unit 130 includes a feature point detecting section 131, as shown in FIG. 3.

Referring to FIG. 3, the feature point detecting section 131 is operable to detect the first feature points of the target object in the reference image. The feature point detecting section 131 is further operable to detect second feature points of the target object in the ARFI image. The first and second feature points include boundary points of the target object. However, the feature points are not limited thereto. Also, the first and second feature points are detected based on a variation of brightness determined by using a differential operator. In one embodiment, the feature point detecting section 131 is configured to detect the feature points by using an edge mask such as Sobel, Prewitt, Robert, Canny and the like. In another embodiment, the feature point detecting section 131 is configured to detect the feature points based on a difference of eigenvalues using a structure tensor.

The processing unit 130 further includes a motion data estimating section 132. The motion data estimating section 132 is operable to identify matching points between the first feature points and the second feature points to estimate motion data based on the identified matching points. In one embodiment, the estimation of the motion data is performed by using various image processing techniques such as an optical flow, a block matching, etc. However, the estimation of the motion data is not limited thereto.

The processing unit 130 further includes a stress data calculating section 133. The stress data calculating section 133 is operable to calculate stress data (hereinafter referred to as "first stress data") of the respective first feature points by using the estimated motion data and an amplitude (or power) of the second ultrasound signals. For example, under the assumption that the target object of a flat shape absorbs ultrasound signals, the stress calculating section 133 is operable to calculate the stress data (F) of the respective first feature points as in the following equation:

$$F = \frac{W_{absorbed}}{c} = \frac{2\alpha I}{c} \quad (1)$$

wherein, $W_{absorbed}$ represents power absorbed by the target object at a predetermined spatial position, c represents a sound velocity in the target object, $\alpha$ represents an absorption coefficient of the target object, and I represents a temporal average intensity at a predetermined point.

The processing unit 130 further includes an interpolating section 134. The interpolating section 134 is operable to interpolate the first stress data to thereby calculate stress data (hereinafter referred to as "second stress data") corresponding to pixels of an elastic image. In one embodiment, the interpolating section 134 is operable to detect two adjacent first feature points at each of the first feature points EP, as shown in FIG. 4. The interpolating section 134 is further operable to interpolate the first stress data at each of the first feature points and the first stress data of the detected two adjacent first feature points to thereby calculate the second stress data corresponding to pixels of the elastic image.

The processing unit 130 further includes an elastic image forming section 135. The elastic image forming section 135 is operable to form the elastic image based on the second stress data provided from the interpolating section 134.

Referring back to FIG. 1, the ultrasound system 100 further includes a display unit 140 configured to display the elastic image provided from the image processing unit 130. The display unit 140 is one of a liquid crystal display, a cathode ray tube, a plate display and the like capable of displaying the elastic image. However, the display unit 140 is not limited thereto. Also, the reference image and the ARFI image are displayed on the display unit 140.

**Claims**

1. An ultrasound system (100), comprising:

an ultrasound data acquisition unit (110) configured to transmit/receive first ultrasound signals to/from a target object to output first ultrasound data; and
an image forming unit (120) configured to form a reference image based on the first ultrasound data
**characterized by** further comprising:

a processing unit (130) connected to the ultrasound data acquisition unit (110) and the image forming unit (120), the processing unit (130) being configured to detect first feature points of the target object in the reference image,
wherein the ultrasound data acquisition unit (110) is further configured to transmit/receive second ultrasound signals for applying an acoustic radiation force impulse, ARFI to/from the target object to output second ultrasound data,
the image forming unit (120) is further configured to form an ARFI image based on the second ultrasound data, and
the processing unit (130) is further configured to detect second feature points of the target object in the ARFI image, calculate first stress data at each of the first feature points based on the first and second feature points, and interpolate the first stress data to calculate second stress data corresponding to pixels of an elastic image, the processing unit being further configured to form the elastic image based on the second stress data, and
wherein the second ultrasound signals are focused on each of the first feature points to perform compression upon the target object.

2. The ultrasound system (100) of Claim 1, wherein the processing unit (130) comprises:

a feature point detecting section (131) configured to detect the first feature points of the target object in the reference image and to detect the second feature points of the target object in the ARFI image;
a motion data estimating section (132) configured to identify matching points between the first feature points and the second feature points and to estimate motion data based on the identified matching points;
a stress data calculating section (133) configured to calculate the first stress data at each of first feature points based on the estimated motion data;
an interpolating section (134) configured to interpolate the first stress data to

calculate the second stress data corresponding to pixels of the elastic image; and

an elastic image forming section (135) configured to form the elastic image based on the second stress data.

3. The ultrasound system (100) of Claim 2, wherein the interpolating section (134) is configured to detect two adjacent first feature points at each of the first feature points and to interpolate the first stress data at each of the feature points and the first stress data at the detected two adjacent first feature points.

4. The ultrasound system (100) of Claim 1, wherein the reference image comprises one of a brightness mode image and a three-dimensional image.

5. A method of forming an elastic image in an ultrasound system (100), comprising:

a) transmitting/receiving by using an ultrasound data acquisition unit (110) within the ultrasound system (100) first ultrasound signals to/from a target object to output first ultrasound data;

b) forming by using an image forming unit (120) within the ultrasound system (100) a reference image based on the first ultrasound data;

c) detecting by using a processing unit (130) within the ultrasound system (100) first feature points of the target object in the reference image;

d) transmitting/receiving by using the ultrasound data acquisition unit (110) within the ultrasound system (100) second ultrasound signals for applying an acoustic radiation force impulse, ARFI to/from the target object to output second ultrasound data, **characterized in that** the second ultrasound signals are focused on each of the first feature points to perform compression upon the target object;

e) forming by using the image forming unit (120) within the ultrasound system (100) an ARFI image based on the second ultrasound data;

f) detecting by using the processing unit (130) within the ultrasound system (100) second feature points of the target object in the ARFI image;

g) calculating by using the processing unit (130) within the ultrasound system (100) stress data at each of the first feature points based on the first and second feature points;

h) interpolating by using the processing unit (130) within the ultrasound system (100) the first stress data to calculate second stress data corresponding to pixels of an elastic image; and

i) forming by using the processing unit (130) within the ultrasound system (100) the elastic image based on the second stress data.

6. The method of Claim 5, wherein the step g) comprises:

g1) identifying matching points between the first feature points and the second feature points;

g2) estimating motion data based on the identified matching points; and

g3) calculating first stress data at each of first feature points based on the estimated motion data.

7. The method of Claim 6, wherein the step h) comprises:

detecting two adjacent first feature points at each of the first feature points; and interpolating the first stress data at each of the feature points and the first stress data at the detected two adjacent first feature points to calculate the second stress data.

8. The method of Claim 5, wherein the reference image comprises one of a brightness mode image and a three-dimensional image.

9. A computer readable medium comprising computer executable instructions configured to perform following acts:

a) transmitting/receiving first ultrasound signals to/from a target object to output first ultrasound data;

b) forming a reference image based on the first ultrasound data;

c) detecting first feature points of the target object in the reference image;

d) transmitting/receiving second ultrasound signals for applying an acoustic radiation force impulse, ARFI to/from the target object to output second ultrasound data, **characterized in that** the second ultrasound signals are focused on each of the first feature points to perform compression upon the target object;

e) forming an ARFI image based on the second ultrasound data;

f) detecting second feature points of the target object in the ARFI image;

g) calculating first stress data at each of the first feature points based on the first and second feature points;

h) interpolating the first stress data to calculate second stress data corresponding to pixels of an elastic image; and

i) forming the elastic image based on the second stress data.

10. The computer readable medium of Claim 9, wherein the act g) comprises:

g1) identifying matching points between the first feature points and the second feature points;

g2) estimating motion data based on the identified matching points; and

g3) calculating first stress data at each of first feature points based on the estimated motion data.

11. The computer readable medium of Claim 9, wherein the act h) comprises:

detecting two adjacent first feature points at each of the first feature points; and
interpolating the first stress data at each of the feature points and the first stress data at the detected two adjacent first feature points to calculate the second stress data.

12. The computer readable medium of Claim 9, wherein the reference image comprises one of a brightness mode image and a three-dimensional image.

**Patentansprüche**

1. Ultraschallsystem (100), welches Folgendes aufweist:

eine Ultraschalldaten-Erlangungseinheit (110), die dafür vorgesehen ist, erste Ultraschallsignale zu bzw. von einem Zielobjekt zu übertragen bzw. zu empfangen, um erste Ultraschalldaten auszugeben; und
eine Bilderzeugungseinheit (120), die dafür vorgesehen ist, ein Referenzbild basierend auf den ersten Ultraschalldaten zu erzeugen,
**gekennzeichnet durch**
eine Verarbeitungseinheit (130), die mit der Erlangungseinheit (110) und der Bilderzeugungseinheit (120) verbunden ist, wobei die Verarbeitungseinheit (130) dafür vorgesehen ist, erste Merkmalspunkte des Zielobjekts in dem Referenzbild zu detektieren,
wobei die Ultraschalldaten-Erlangungseinheit (110) des Weiteren dafür vorgesehen ist, zweite Ultraschallsignale zu bzw. von dem Zielobjekt zu übertragen bzw. zu empfangen, um einen akustischen Strahlungskraftimpuls, ARFI, aufzubringen, um zweite Ultraschalldaten auszugeben,
wobei die Bilderzeugungseinheit (120) des Weiteren dafür vorgesehen ist, ein ARFI-Bild basierend auf den zweiten Ultraschalldaten zu erzeugen, und
wobei die Verarbeitungseinheit (130) des Weiteren dafür vorgesehen ist, zweite Merkmalspunkte des Zielobjekts in dem ARFI-Bild zu detektieren, erste Beanspruchungsdaten an jedem der ersten Merkmalspunkte basierend auf den ersten und zweiten Merkmalspunkten zu berechnen und die ersten Beanspruchungsdaten zu interpolieren, um zweite Beanspruchungssdaten entsprechend den Pixeln eines elastischen Bilds zu berechnen, wobei die Verarbeitungeinheit des Weiteren dafür vorgesehen ist, das elastische Bild basierend auf den zweiten Beanspruchungsdaten zu erzeugen, und
wobei die zweiten Ultraschallsignale auf jeden der ersten Merkmalspunkte fokussiert sind, um eine Kompression an dem Zielobjekt durchzuführen.

2. Ultraschallsystem (100) nach Anspruch 1, wobei die Verarbeitungseinheit (130) Folgendes aufweist:

einen Merkmalspunkt-Detektierabschnitt (131), der dafür vorgesehen ist, die ersten Merkmalspunkte des Zielobjekts in dem Referenzbild zu detektieren und die zweiten Merkmalspunkte des Zielobjekts in dem ARFI-Bild zu detektieren;
einen Bewegungsdaten-Bewertungsabschnitt (132), der dafür vorgesehen ist, übereinstimmende Punkte zwischen den ersten Merkmalspunkten und den zweiten Merkmalspunkten zu identifizieren und Bewegungsdaten basierend auf den identifizierten übereinstimmenden Punkten zu bewerten;
einen Beanspruchungsdaten-Berechnungsabschnitt (133), der dafür vorgesehen ist, die ersten Beanspruchungsdaten an jedem der ersten Merkmalspunkte basierend auf den bewerteten Bewegungsdaten zu berechnen;
einen Interpolierabschnitt (134), der dafür vorgesehen ist, die ersten Beanspruchungsdaten zu interpolieren, um die zweiten Beanspruchungsdaten, die den Pixeln des elastischen Bilds entsprechen, zu berechnen; und
einen Abschnitt (135) zur Erzeugung eines elastischen Bilds, der dafür vorgesehen ist, das elastische Bild basierend auf den zweiten Beanspruchungsdaten zu erzeugen.

3. Ultraschallsystem (100) nach Anspruch 2, wobei der Interpolierabschnitt (134) dafür vorgesehen ist, zwei benachbarte erste Merkmalspunkte an jedem der ersten Merkmalspunkte zu detektieren und die ersten Beanspruchungsdaten an jedem der Merkmalspunkte und die ersten Beanspruchungsdaten an den detektierten zwei benachbarten ersten Merkmalspunkten zu identifizieren.

4. Ultraschallsystem (100) nach Anspruch 1, wobei das Referenzbild ein Helligkeitsmodusbild oder ein dreidimensionales Bild aufweist.

5. Verfahren zur Erzeugung eines elastischen Bilds in einem Ultraschallsystem (100), welches Folgendes aufweist:

a) Übertragen bzw. Empfangen von ersten Ultraschallsignalen zu bzw. von einem Zielobjekt unter Verwendung einer Ultraschalldaten-Erlangungseinheit (110) innerhalb des Ultraschallsystems (100), um erste Ultraschalldaten auszugeben;
b) Erzeugen eines Referenzbilds basierend auf den ersten Ultraschalldaten unter Verwendung einer Bilderzeugungseinheit (120) innerhalb des Ultraschallsystems (100),
c) Detektieren erster Merkmalspunkte des Zielobjekts in dem Referenzbild unter Verwendung einer Verarbeitungseinheit (130) innerhalb des Ultraschallsystems (100),
d) Übertragen bzw. Empfangen zweiter Ultraschallsignale unter Verwendung der Ultraschalldaten-Erlangungseinheit (110) innerhalb des Ultraschallsystems (100) zur Aufbringung bzw. Anwendung eines akustischen Strahlungskraftimpulses, ARFI zu bzw. von dem Zielobjekt, um zweite Ultraschalldaten auszugeben, **dadurch gekennzeichnet, dass** die zweiten Ultraschalldaten auf jeden der ersten Merkmalspunkte fokussiert werden, um eine Kompression an dem Zielobjekt durchzuführen;
e) Erzeugen eines ARFI-Bilds basierend auf den zweiten Ultraschalldaten unter Verwendung der Bilderzeugungseinheit (120) innerhalb des Ultraschallsystems (100);
f) Detektieren zweiter Merkmalspunkte des Zielobjekts in dem ARFI-Bild unter Verwendung der Verarbeitungseinheit (130) innerhalb des Ultraschallsystems (100);
g) Berechnen von Beanspruchungsdaten an jedem der ersten Merkmalspunkte basierend auf den ersten und zweiten Merkmalspunkten unter Verwendung der Verarbeitungseinheit (130) innerhalb des Ultraschallsystems (100);
h) Interpolieren der ersten Beanspruchungsdaten, um zweite Beanspruchungsdaten zu berechnen, die Pixeln eines elastischen Bilds entsprechen, unter Verwendung der Verarbeitungseinheit (130) innerhalb des Ultraschallsystems (100); und
i) Erzeugen des elastischen Bilds basierend auf den zweiten Beanspruchungsdaten unter Verwendung der Verarbeitungseinheit (130) innerhalb des Ultraschallsystems (100).

6. Verfahren nach Anspruch 5, wobei der Schritt g) Folgendes aufweist:

g1) Identifizieren einander entsprechender bzw. übereinstimmender Punkte zwischen den ersten Merkmalspunkten und den zweiten Merkmalspunkten;
g2) Bewerten von Bewegungsdaten basierend auf den identifizierten übereinstimmenden Punkten; und
g3) Berechnen erster Beanspruchungsdaten an jedem der ersten Merkmalspunkte basierend auf den bewerteten Bewegungsdaten.

7. Verfahren nach Anspruch 6, wobei der Schritt h) Folgendes aufweist:

Detektieren zweier benachbarter erster Merkmalspunkte an jedem der ersten Merkmalspunkte; und
interpolieren der ersten Beanspruchungsdaten an jedem der ersten Merkmalspunkte und der ersten Beanspruchungsdaten an den detektierten zwei benachbarten ersten Merkmalspunkten, um die zweiten Beanspruchungsdaten zu berechnen.

8. Verfahren nach Anspruch 5, wobei das Referenzbild ein Helligkeitsmodusbild oder ein dreidimensionales Bild aufweist.

9. Computerlesbares Medium, das von einem Computer ausführbare Anweisungen aufweist, und das dafür vorgesehen ist, die folgenden Schritte vorzunehmen:

a) Übertragen bzw. Empfangen von ersten Ultraschallsignalen zu bzw. von einem Zielobjekt, um erste Ultraschalldaten auszugeben;
b) Erzeugen eines Referenzbilds basierend auf den ersten Ultraschalldaten;
c) Detektieren erster Merkmalspunkte des Zielobjekts in dem Referenzbild;
d) Übertragen bzw. Empfangen zweiter Ultraschallsignale zu bzw. von dem Zielobjekt, um einen akustischen Strahlungskraftimpuls, ARFI anzuwenden, **dadurch gekennzeichnet, dass** die zweiten Ultraschallsignale auf jeden der ersten Merkmalspunkte fokussiert werden, um eine Kompression an dem Zielobjekt durchzuführen;
e) Erzeugen eines ARFI-Bilds basierend auf den zweiten Ultraschalldaten;
f) Detektieren zweiter Merkmalspunkte des Zielobjekts in dem ARFI-Bild;
g) Berechnen erster Beanspruchungsdaten an jedem der ersten Merkmalspunkte basierend auf den ersten und zweiten Merkmalspunkten;
h) Interpolieren der ersten Beanspruchungsdaten, um zweite Beanspruchungsdaten zu berechnen, die Pixel eines elastischen Bilds entsprechen; und
i) Erzeugen des elastischen Bilds basierend auf den zweiten Beanspruchungsdaten.

10. Computerlesbares Medium nach Anspruch 9, wobei der Schritt g) Folgendes aufweist:

g1) Identifizieren einander entsprechender bzw. übereinstimmender Punkte zwischen den ersten Merkmalspunkten und den zweiten Merkmalspunkten;
g2) Bewerten von Bewegungsdaten basierend auf den identifizierten übereinstimmenden Punkten; und
g3) Berechnen erster Beanspruchungsdaten an jedem der ersten Merkmalspunkte basierend auf den bewerteten Bewegungsdaten.

11. Computerlesbares Medium nach Anspruch 9, wobei der Schritt h) Folgendes aufweist:

Detektieren zweier benachbarter erster Merkmalspunkte an jedem der ersten Merkmalspunkte; und
Interpolieren der ersten Beanspruchungsdaten an jedem der ersten Merkmalspunkte und der ersten Beanspruchungsdaten an den detektierten zwei benachbarten ersten Merkmalspunkten, um die zweiten Beanspruchungsdaten zu berechnen.

12. Computerlesbares Medium nach Anspruch 9, wobei das Referenzbild ein Helligkeitsmodusbild oder ein dreidimensionales Bild aufweist.

**Revendications**

1. Système ultrasonique (100) comportant :

une unité d'acquisition de données ultrasoniques (110) configurée pour transmettre/recevoir des premiers signaux ultrasoniques vers/provenant d'un objet cible pour émettre des premières données ultrasoniques ; et une unité de formation d'image (120) configurée pour former une image de référence basée sur les premières données ultrasoniques,
**caractérisé en ce qu'**il comporte en outre :

une unité de traitement (130) connectée à l'unité d'acquisition de données ultrasoniques (110) et à l'unité de formation d'image (120), l'unité de traitement (130) étant configurée pour des premiers points objets de l'objet cible dans l'image de référence,
dans lequel l'unité d'acquisition de données ultrasoniques (110) est en outre configurée pour transmettre/recevoir des seconds signaux ultrasoniques pour appliquer une impulsion de force par rayonnement acoustique ARFI vers/de l'objet cible pour émettre des secondes données ultrasoniques,
l'unité de formation d'image (120) étant en outre configurée pour former une image ARFI basée sur les secondes données ultrasoniques, et
l'unité de traitement (130) étant en outre configurée pour détecter des seconds points image de l'objet cible de l'image ARFI, pour calculer des premières données de tension en chacun des premiers points objets basées sur les premiers et seconds points objets, et pour insérer les premières données de tension afin de calculer les secondes données de tension correspondant aux pixels d'une image élastique, l'unité de traitement étant en outre configurée pour former l'image élastique basée sur les secondes données de tension, et
dans lequel, les seconds signaux ultrasoniques sont focalisés sur chacun des points image pour effectuer une compression sur l'objet cible.

2. Système ultrasonique (100) selon la revendication 1, dans lequel l'unité de traitement (130) comprend :

une section (131) de détection de points objet configurée pour détecter les premiers points objet de l'objet cible dans l'image de référence et pour détecter les seconds points objet de l'objet cible de l'image ARFI ;
une section d'estimation de données de mouvements (132) configurée pour identifier les points identiques entre les premiers points objet et les seconds points objet et pour estimer les données de mouvements basées sur les points identiques identifiés ;
une section de calcul des tensions (133) configurée pour calculer les premières données de tensions de chacun des points objet basées sur les données de mouvement estimées ;
une section d'interpolation (134) configurée pour insérer les premières données de tension en vue de calculer les secondes données de tension correspondant aux pixels de l'image élastique ; et
une section de formation d'image élastique (135) configurée pour former une image élastique basée sur les secondes données de tension.

3. Système ultrasonique (100) selon la revendication 2, dans lequel l'unité d'interpolation (134) est en outre configurée pour détecter deux premiers points objet adjacents pour chacun des premiers points objet et pour insérer les premières données de tension de chacun des points objet et les premières données de tension aux deux premiers points objet adjacents détectés.

4. Système ultrasonique (100) selon la revendication 1, dans lequel l'image de référence comprend un des modes de brillance d'image et une image tridimensionnelle.

5. Procédé de formation d'une image élastique dans un système ultrasonique (100) comportant :

a) la transmission/réception en utilisant une unité d'acquisition de données ultrasoniques (110) du système ultrasonique (100) envoyés/reçus d'un objet cible parmi les premiers signaux ultrasoniques pour émettre des premières données ultrasoniques ;

b) la formation d'une image de référence basée sur les premières données ultrasoniques en utilisant l'unité de formation d'image (120) du système ultrasonique (100)

c) la détection de points objet de l'objet cible de l'image de référence, en utilisant une unité de traitement (130) du système ultrasonique (100);

d) la transmission/réception en utilisant l'unité d'acquisition de données ultrasoniques (110) du système ultrasonique (100) de seconds signaux ultrasoniques pour appliquer une impulsion de force par radiation acoustique ARFI vers/de l'objet cible pour émettre des secondes données ultrasoniques, **caractérisé en ce que** les seconds signaux ultrasoniques sont focalisés sur chacun des premiers points objet pour effectuer une compression sur l'objet cible ;

e) la formation en utilisant l'unité de formation (120) du système ultrasonique (100) d'une image ARFI basée sur les secondes données ultrasoniques ;

f) la détection en utilisant l'unité de traitement (130) du système ultrasonique (100) de seconds points objet de l'objet cible de l'image ARFI ;

g) le calcul en utilisant l'unité de traitement (130) du système ultrasonique (100) de données de tension de chacun des premiers points objet basés sur les premiers et les seconds points objet ;

h) l'interpolation en utilisant l'unité de traitement (130) du système ultrasonique (100) des premières données de tension correspondant à des pixels d'une image élastique ; et

i) la formation en utilisant l'unité de traitement (130) du système ultrasonique (100) de l'image élastique basée sur les secondes données de tension.

6. Procédé selon la revendication 5, dans lequel l'étape g) comprend :

g1) l'identification de points identiques entre les premiers et les seconds points objet ;

g2) l'estimation des données de mouvement sur les points identiques identifiés ; et

g3) le calcul de données de tension de chacun des premiers points objet basés sur les données de mouvement estimées.

7. Procédé selon la revendication 6, dans lequel l'étape h) comprend :

la détection de deux premiers points objet adjacents en chacun des premiers points objet et l'interpolation des premières données de tension en chacun des points objet et des premières données de tension aux deux premiers points objet adjacents détectés.

8. Procédé selon la revendication 5, dans lequel l'image de référence comporte un des modes de brillance d'image et une image tridimensionnelle.

9. Support lisible par ordinateur comportant des instructions pouvant être exécutées par un ordinateur pour effectuer les opérations suivantes :

a) la transmission/réception des premiers signaux ultrasoniques envoyés/reçus d'un objet cible pour émettre des premières données ultrasoniques ;

b) la formation d'une image de référence basée sur les premières données ultrasoniques ;

c) la détection de premiers points objet de l'objet cible de l'image de référence ;

d) la transmission/réception de seconds signaux ultrasoniques pour appliquer une impulsion de force par radiation acoustique ARFI vers/de l'objet cible pour émettre des secondes données ultrasoniques, **caractérisé en ce que** les seconds signaux ultrasoniques sont focalisés sur chacun des premiers points objet pour effectuer une compression sur l'objet cible ;

e) la formation d'une image ARFI basée sur les secondes données ultrasoniques ;

f) la détection de seconds points objet de l'objet cible de l'image ARFI ;

g) le calcul de données de tension de chacun des premiers points objet basées sur les premiers et les seconds points objet ;

h) l'interpolation des premières données de tension correspondant à des pixels d'une image élastique ; et

i) la formation de l'image élastique basée sur les secondes données de tension.

10. Support lisible par ordinateur selon la revendication 9, dans lequel l'étape g) comprend :

g1) l'identification de points identiques entre les premiers et les seconds points objet ;

g2) l'estimation des données de mouvement sur les points identiques identifiés ; et

g3) le calcul de données de tension de chacun des premiers points objet basés sur les données de mouvement estimées.

**11.** Support lisible par ordinateur selon la revendication 9, dans lequel l'étape h) comprend :

la détection de deux premiers points objet adjacents en chacun des premiers points objet et l'interpolation des premières données de tension en chacun des points objet et des premières données de tension aux deux premiers points objet adjacents détectés.

**12.** Support lisible par ordinateur selon la revendication 9, dans lequel l'image de référence comprend un des modes de brillance d'image et une image tridimensionnelle.

# FIG. 1

<u>100</u>

120

IMAGE
FORMING
UNIT

110

ULTRASOUND DATA
ACQUISITION UNIT

130

PROCESSING
UNIT

140

DISPLAY
UNIT

# FIG. 2

<u>110</u>

111

Tx SIGNAL
GENEREATING
SECTION

ULTRASOUND
PROBE

BEAM FORMER

ULTRASOUND
DATA FORMING
SECTION

112

113

114

# FIG. 3

130

| 131 | 132 | 133 | 134 | 135 |
|---|---|---|---|---|
| FEATURE POINT DETECTING SECTION | MOTION DATA ESTIMATING SECTION | STRESS DATA CALCULATING SECTION | INTERPOLATING SECTION | ELASTIC IMAGE FORMING SECTING |

# FIG. 4

210

EP

EP

EP

EP

EP

EP

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2899336 A1
- EP 1520517 A1
- US 2007073145 A1
- US 6558324 B1

**Non-patent literature cited in the description**

- **DOUGLAS DUMONT.** *ARFI imaging for noninvasive material characterization of atherosclerosis*
- **R.H. BEHLER.** *ARFI ultrasound for characterizing atherosclerosis*
- **R.H. BEHLER.** *Acoustic radiation force impulse imaging of the abdomen: Demonstration of feasibility and utility*